# EUROPEAN PATENT APPLICATION

(11) **EP 1 155 655 A1**
(43) Date of publication of application: **21.11.2001**
(21) Application number: 00110748.1
(22) Date of filing: 19.05.2000
(51) Int. Cl.: A61B 1/253

(54) **An odontoscope and the related equipment thereof**

(71) Applicant: Lu, Kuo-Chuan, Taipei Hsien, Taiwan, R.O.C. (TW); Lu, Kuo-Hsun, Taipei Hsien, Taiwan, R.O.C. (TW)
(72) Inventor: Lu, Kuo-Chuan, Taipei Hsien, Taiwan, R.O.C. (TW); Lu, Kuo-Hsun, Taipei Hsien, Taiwan, R.O.C. (TW)
(74) Representative: Dallmeyer, Georg, Dipl.-Ing.

(57) **Abstract**

An odontoscope and the related equipment thereof are provided. The odontoscope mainly includes a hollow long handle and a mirror frame that is connected to a front end of the hollow long handle with a reflecting mirror firmly fitted therein. The hollow long handle defines a tubular space therein and having an air jetting port provided close to the front end thereof to supply jetted air toward the reflecting mirror at an angle of 135 degrees relative to the reflecting mirror. A rear end of the hollow long handle is connected to a flexible tube that is in turn connected to an air compressor. When the air compressor is started to run, an airflow is supplied from it to pass the tube and the hollow long handle to jet from the air jetting port, so that any residuum, including mist, saliva and ground or drilled tooth debris, on said reflecting mirror can be quickly cleaned out by the jetted air.

## Description

### BACKGROUND OF THE INVENTION:

The present invention relates to an odontoscope and the related equipment thereof, and more particularly to an odontoscope that are provided with means to effectively clean mist, saliva, and ground or drilled tooth debris from a reflecting mirror on the odontoscope, so that a dentist may efficiently utilize the reflecting mirror to visual examine a patient's teeth and do correct diagnosis and treatment.

people use their teeth to eat everyday and tend to suffer from different dental diseases, including periodontitis, carious teeth, etc., and grinding toothache, if they did not well protect their teeth through good dental hygiene. Dental diseases and toothache require examination and treatment by a dentist. The dentist would normally visually examine and treat the patient's oral cavity and teeth with the help of an odontoscope. The odontoscope includes a reflecting mirror. Reflection in the mirror enables the dentist to explore and correctly judge the conditions in the patient's oral cavity before doing any suitable diagnosis and treatment.

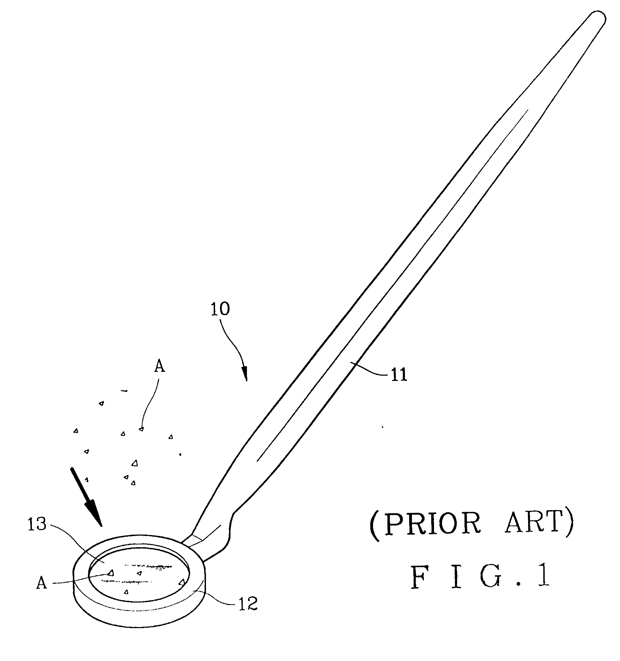

Fig. 1 is a perspective of a conventional odontoscope (10) that generally includes a solid handle (11), a mirror frame (12) fixedly connected to a front end of the solid handle (11), and a reflecting mirror (13) fitted in the mirror frame (12). When the dentist extends the reflecting mirror (13) of the odontoscope (10) into the patient's oral cavity for the purpose of examining the patient's teeth, the reflecting mirror (13) tends to be quickly covered with a thin layer of mist due to the patient's breath and becomes vague that would obviously adversely affect the dentist's diagnosis and treatment. And, when the dentist treats the patient's teeth by, for example, grinding or drilling the teeth with some grinder or drill, tooth debris (A) from such grinding and drilling as well as a large amount of saliva would constantly accumulate on the reflecting mirror (13) to cover the same and seriously prevent the dentist from examining or treating the teeth successfully. To cope with this problem,the dentist must repeatedly remove the reflecting mirror (13) of the odontoscope (10) from the patient's oral cavity to clean out the mist, saliva and tooth debris on the reflecting mirror (13). This is, of course, very inconvenient to the dentist and would even result in interrupted, inefficient and incorrect diagnosis and treatment. Meanwhile, such interrupted treatment also makes the patient nervous.

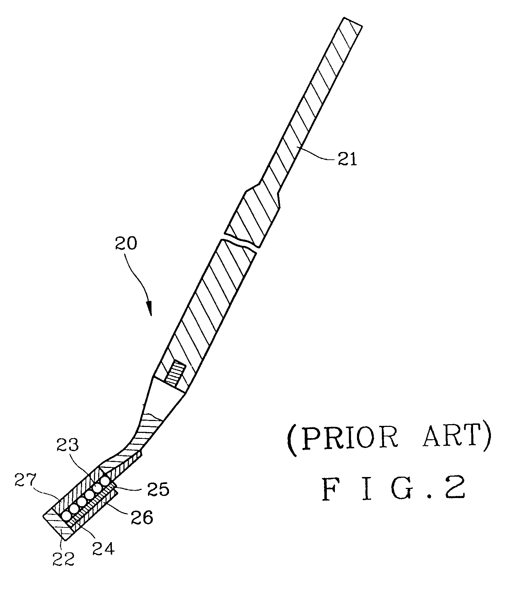

To eliminate the above-mentioned disadvantages in the conventional odontoscope (10), another odontoscope (20) as shown in Fig.2 was developed. The odontoscope (20) also includes substantially a handle (21) and a mirror holder (22) connected to a front end of the handle (21). The mirror holder (22) is provided with a recess (24) into which heat-absorbing substance (23) is the form of granules are packed. The heat-absorbing substance (23) may be zeolite, caustic lime, active aluminum oxide, slaked lime, etc. A cover (25) and a sealing die (26) are fixedly provided at a bottom of the recess (24), and a reflecting mirror (27) is fitted on a top of the mirror holder (22) with a backside of the mirror (27) directly contacting with the heat-absorbing granules (23). Whereby, any mist formed on the reflecting mirror (27) can be completely absorbed by the heat-absorbing granules (23) to avoid a vague reflecting mirror (27) and therefore facilitate smooth and correct diagnosis and treatment by the dentist. A disadvantage of this type of conventional odontoscope (20) is that it is effective only in removing mist and saliva on the reflecting mirror (27), but not the ground or drilled tooth debris that would still cause problems in the effective use of the odontoscope (20).

### SUMMARY OF THE INVENTION:

It is therefore a primary object of the present invention to provide an odontoscope that is provided with means to effectively clean out any mist, saliva, and ground or drilled tooth debris accumulated on the reflecting mirror of the odontoscope at any time, so that diagnosis and treatment done by a dentist with the odontoscope would not be adversely affected.

To achieve the above and other object, the present invention mainly includes an odontoscope, a flexible tube, and an external air compressor. The odontoscope includes a hollow long handle and a mirror frame that is connected to a front end of the hollow long handle with a reflecting mirror firmly fitted therein. The hollow long handle defines a tubular space therein and having an air jetting port provided close to the front end thereof to supply jetted air toward the reflecting mirror at an angle of 135 degrees. A rear end of the hollow long handle is connected to the flexible tube that is in turn connected to the air compressor. When the air compressor is started to run, an airflow is supplied from it to pass the tube and the hollow long handle to jet from the air jetting port, so that any residuum, including mist, saliva and grpund or drilled tooth debris, on said reflecting mirror can be quickly cleaned out.

### BRIEF DESCRIPTION OF THE DRAWINGS:

The structure and the technical means adopted by the present invention to achieve the above and other objects can be best understood by referring to the following detailed description of the preferred embodiments and the accompanying drawings, wherein
Fig. 1 is a perspective of a first conventional odontoscope;
Fig. 2 is a side sectional view of another conventional odontoscope;
Fig. 3 shows an odontoscope and the related equipment thereof according to the present invention; and
Fig. 4 is a side sectional view of the odontoscope of Fig. 3 showing the operation thereof.

### DETAILED DESCRIPTION OF THE PREFERRED EMBODIMENTS:

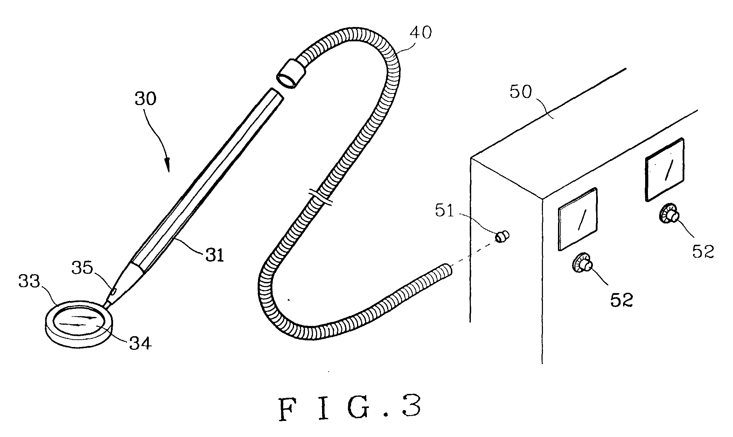

Please refer to Fig. 3 in which an odontoscope (30) and the related equipment thereof according to the present invention are shown. The equipment related to the odontoscope (30) mainly includes a tube (40) and an air compressor (50) that are externally connected to the odontoscope (30) for supplying air to the latter. The air compressor (50) may be one that has been originally included in a dental unit (not shown), and it is not necessary to separately or additionally purchase and mount an air compressor for the operation of the odontoscope (30) of the present invention.

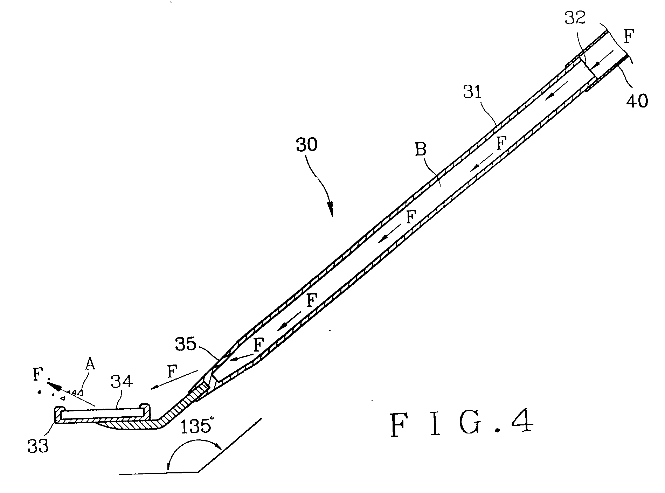

Please now refer to Figs.3 and 4 at the same time. The odontoscope (30) mainly includes a hollow long handle (31) and a mirror frame (33) that is connected to a front end of the hollow long handle (31) with a reflecting mirror (34) firmly fitted in the mirror frame (33). The hollow long handle (31) defines a tubular space (B) therein. A rear end of the hollow long handle (31) opposite to the front end having the reflecting mirror (34) defines an air inlet (32). And, an air jetting port (35) is provided close to the front end of the hollow long handle (31) to face a top surface of the reflecting mirror (34). The air jetting port (35) is so oriented that air is jetted from the port (35) toward the reflecting mirror (34) at an angle of 135 degrees relative to the reflecting mirror (34).

The tube (40) is a flexible tube made of a material with sufficient elasticity and rigidity. An end of the tube (40) is connected to the air inlet (32) at the rear end of the hollow long handle (31), and the other end of the tube (40) is connected to an air outlet (51) provided on the air compressor (50).

To use the odontoscope (30) of the present invention in a dental surgery or tooth treatment, first connect a predetermined end of the tube (40) to the air inlet (32) at the rear end of the hollow long handle (31) and another end of the tube (40) to the air outlet (51) of the air compressor (50). When the air compressor (50) is started to run, an airflow (F) is supplied from the air compressor (50) to flow through the tube (40) and into the tubular space (B) of the hollow long handle (31). The airflow (F) is finally jetted from the air jetting port (35) close to the front end of the hollow long handle (31) to powerfully blow against the reflecting mirror (34) at an angle of 135 degrees relative to the reflecting mirror (34). This angle of 135 degrees is an optimum refracting angle for the airflow blown against the reflecting mirror (34) to quickly clean out any residuum (A) on the reflecting mirror (34), such as mist, saliva, and debris of ground or drilled teeth, so that the dental surgery would not be undesirably stopped.

The air compressor (50) may be provided with an automatic timer (52) that could be set or controlled by the dentist to decide duration and interval of supplying jetted airflow from the air compressor (50) to the reflecting mirror (34) via the air jetting port (35).

The odontoscope (30) of the present invention and the related equipment thereof have simple structure but they do help a dentist complete the tooth diagnosis and treatment efficiently and conveniently and are therefore practical and improved medical equipment for use.

## Claims

1. An odontoscope and the related equipment thereof, comprising an odontoscope, a tube, and an air compressor;
said odontoscope including a hollow long handle, a mirror frame fixedly connected to a front end of said hollow long handle, and a reflecting mirror firmly fitted in said mirror frame; said hollow long handle defining a tubular space therein and having an air jetting port provided close to said front end of said hollow long handle (31) to face a top surface of said reflecting mirror;
said tube being a flexible tube made of a material with sufficient elasticity and rigidity; an end of said tube being connected to an air inlet provided at a rear end of said hollow long handle, and the other end of said tube being connected to an air outlet provided on said air compressor; and
said air compressor being provided with an automatic timer for controlling the supply of airflow from said air compressor to said reflecting mirror via said tube and said hollow long handle at preset duration and intervals;
Whereby when said air compressor is started to run, an airflow is supplied from said air compressor to pass said tube and said hollow long handle and finally jet from said air jetting port close to said front end of said hollow long handle to quickly clean out any residuum, including mist, saliva and ground or drilled tooth debris on said reflecting mirror.

2. An odontoscope and the related equipment thereof as claimed in claim 1, wherein said air jetting port is so oriented that air is jetted from said port toward said reflecting mirror at an angle of 135 degrees relative to said reflecting mirror.
